# EUROPEAN PATENT SPECIFICATION

(11) **EP 1 669 058 B1**
(45) Date of publication and mention of the grant of the patent: **26.05.2010**
(21) Application number: 04425820.0
(22) Date of filing: 03.11.2004
(51) Int. Cl.: A61K 8/22, A61K 8/25, A61Q 5/08

(54) **Bleaching powder for keratinic fibers**
Pulver zur Entfärbung von keratinischen Fasern
Poudre pour la décoloration des fibres kératiniques

(43) Date of publication of application: 14.06.2006
(73) Proprietor: Farmen International Cosmetics Distribution S.p.A, 10036 Settimo Torinese (TO) (IT)
(72) Inventor: Manzetti, Gianpiero c/o Farmen S.P.A., 10036 Settimo Torinese (Torino) (IT)
(74) Representative: Freyria Fava, Cristina

(56) References cited:
- EP-A- 0 583 767
- EP-A- 1 213 009
- EP-A- 1 258 237
- GB-A- 1 275 173
- US-A- 5 783 175

## Description

### TEXT OF THE DESCRIPTION

The present invention relates to a bleaching powder for keratinic fibres. In particular, the present invention relates to a composition in the form of anhydrous powder for bleaching human keratinic fibres, more specifically hair.

For bleaching keratinic fibres and, in particular, hair, bleaching powders are generally used which contain peroxide-based compounds, amongst which it is possible, for example, to cite persulphates, percarbonates or perborates of ammonium or of alkaline metals, to which alkalinizing compounds are generally associated - also referred to as activating agents - amongst which, by way of example, it is possible to mention silicates, metasilicates, phosphates or carbonates of alkaline metals or else ammonium salts.

Said powders are, at the moment of use, mixed with hydrogen-peroxide-based acidic aqueous emulsions. The presence of the alkalinizing compounds determines a shift of the pH of the mixture from acidic to basic, with consequent release of active oxygen (the bleaching agent) both on the part of the peroxide-based compounds and on the part of the hydrogen peroxide itself, which are unstable in conditions of a basic pH.

A common drawback of said bleaching powders is determined by their high powderiness, i.e., by the release, during the stage of preparation of the mixture by the user and during the stage of fabrication, of very fine powders containing substantially peroxide-based compounds that prove very dangerous and harmful for the operators, especially if they are inhaled.

This drawback has been tackled in the known art by adding to the basic mixture in powder form agents capable of binding the particles of powder, so reducing their volatility.

The patent application No. EP-A-0 560 088 envisages adding to the bleaching preparation in powder form oils or liquid waxes that are not sensitive to processes of oxidation and are capable of incorporating the particles of powder. Said compounds are, however, difficult to disperse in the preparation and are highly inflammable, in addition to being very difficult to remove from the hair at the end of treatment.

Other patent documents envisage, instead, adding anhydrous polymeric compounds, soluble in an aqueous solution such as polyethylene glycols or polypropylene glycols (FR-A-2 716 804), or else co-polymers of polyoxyethylene and polyoxypropylene (EP-A-0 663 205).

In the patent application No. EP-A-1 228 751, it is proposed to add non-ionic and/or anionic amphyphilic polymers, preferably derivatives of methacrylic acid, bound to a fatty chain.

Other solutions proposed in the past envisage the use of processes applied in the pharmaceutical industry, such as wet granulation of the powders as in EP-A-0 574 696, where there are used as binding compounds, hence ones capable of compacting the powders into granules of larger dimensions, a co-polymer of methacrylic acid and methylmethacrylate in the presence of sodium hydroxide and water.

The mixture of the bleaching powder with the oxidizing emulsion must moreover meet other requirements: 1) possess a consistency such as to enable its application on hair in the absence of dripping; and 2) adhere to the hair up to the end of the treatment, which usually lasts from 30 to 60 minutes.

A bleaching powder with the aforesaid characteristics is known from the patent application No. FR-A-2 788 976. Said application envisages the addition to the bleaching powder, comprising peroxide-based compounds and alkalinizing agents, of gelling agents (derivatives of cellulose, starch, alginates, or silica) together with water-soluble polymers of natural or synthetic origin which, in combination with at least one non-ionic and/or anionic amphyphilic polymer, provide the mixture with greater consistency.

A purpose of the present invention is to provide a bleaching powder that will overcome more effectively the drawbacks referred to above, and that will reduce the intrinsic inflammability of said powders determined principally by the presence of peroxide-based compounds and moreover increase the stability of storage of the bleaching powder even for long periods of time.

A further purpose of the bleaching powder forming the subject of the present invention is the absence of a volumetric increase during the development of oxygen of the mixture obtained from the mixing of the anhydrous powder with the hydrogen-peroxide-based aqueous emulsion when it is used on the hair.

According to the present invention, said purposes are achieved by a bleaching powder for keratinic fibres having the characteristics recalled in a specific way in the ensuing claims.

More specifically, the bleaching powder forming the subject of the present invention comprises at least one peroxide-based compound, at least one alkalinizing agent, and a superabsorbent material. The bleaching powder forming the subject of the present invention can moreover comprise at least one gelling agent.

The use of said superabsorbent material in a powder composition for the bleaching of hair has surprisingly revealed the capacity of said material to reduce the powderiness of the powder composition.

It has moreover been surprisingly noted that said superabsorbent material is able to reduce the reactivity of the powder composition, and, in particular, to reduce to a minimum the risks of inflammability intrinsic in the powder composition and basically determined by the presence of peroxide-based compounds. Said compounds can in fact be inflamed by mere rubbing during the step of industrial preparation, as well as on account of an excessive humidity of the environment in which the process is being carried out, or else on account of high environmental temperatures.

A further advantage linked to the use of a superabsorbent material within a bleaching composition for keratinic fibres in powder form is bound to an increase of the stability of storage of the composition itself. Without wishing to be tied down to a specific theory in this regard, the present applicant has in fact reason to believe that said compound will reduce the decay in time of the titre of active oxygen.

For a better understanding of the present invention, there is now described a preferred embodiment thereof, which is provided purely by way of non-limiting example.

The bleaching powder for keratinic fibres according to the present invention comprises at least one peroxide-based compound, at least one alkalinizing agent and a superabsorbent material which is a Superabsorbent Polymer (SAP) constituted by cross-linked sodium or potassium polymethacrylate. The bleaching powder may moreover comprise at least one gelling agent.

The superabsorbent material used in the context of the present invention is substantially constituted by an appropriately cross-linked methacrylic-based polymer; said polymer may moreover advantageously assume a salified form, preferably with an alkaline metal.

Said superabsorbent material, in addition to solving to an unexpected extent all the drawbacks linked to the use of these bleaching preparations, enables: 1) reduction of the inflammability of the peroxide-based compounds; 2) reduction of the reactivity of the powder when brought into contact with water or high environmental humidity; 3) increase of the stability of storage of the powder in time; and 4) solution also of the problem linked to the maintenance of the correct viscosity of the mixture during the application on the hair.

It has in fact been observed that the reduction of the reactivity of the bleaching powder can readily be verified by progressive addition of water up to a value corresponding to 10 wt%. The temperature of the mixture never exceeds 50°C, whilst in the absence of said superabsorbent material the temperature of the mixture frequently reaches 110°C.

Purely by way of illustration, Table 1 reproduces the composition of a bleaching powder according to the present invention.

**Table 1**

| Ingredient | wt% with respect to the total weight of the powder |
|---|---|
| Peroxide-based compound(s) | 40 - 60 |
| Alkalinizing compound(s) | 5 - 15 |
| Superabsorbent material | 0.5 - 8 |
| Gelling compound(s) | 0.5 - 4 |
| Additives | 0 - 5 |

Amongst the peroxide-based compounds persulphate-based compounds are preferred, such as ammonium persulphate, sodium persulphate, or potassium persulphate; as alkalinizing compounds it is possible to cite sodium silicate, sodium metasilicate, and sodium carbonate. As regards gelling compounds the following are preferred: guar gum, xanthan gum, polymethacrylates (carbomers).

The additives usually employed are sequestering agents, lubricants, and other compounds commonly known to the person skilled in the branch.

The superabsorbent material used in the bleaching powder forming the subject of the present invention is a cross-linked polymer of sodium or potassium polymethacrylate, synthesized in an aqueous environment, insoluble in water with a capacity for absorption of water of up to 300 times its own weight (for example, the SAP Luquasorb 1010, distributed by BASF).

A preferred composition of a bleaching powder according to the present invention is reproduced in Table 2.

**Table 2**

| Ingredient | wt% with respect to the total weight of the powder |
|---|---|
| Ammonium persulphate | 20 - 30 |
| Potassium persulphate | 20 - 30 |
| Sodium silicate | 10 - 20 |
| Sodium metasilicate | 5 - 15 |
| Magnesium carbonate | 5 - 20 |
| Guar gum | 1 - 3 |
| Sodium stearate | 1 - 5 |
| Tetrasodium EDTA | 1 - 3 |
| Superabsorber (SAP) | 0.5 - 8 |

## Claims

1. An anhydrous powder composition for bleaching keratinic fibres, in particular human keratinic fibres, comprising at least one peroxide-based compound and at least one alkalinizing agent, **characterized in that** it further comprises a superabsorbent polymer wherein
said superabsorbent polymer is a polymeric compound constituted by cross-linked sodium or potassium polymethacrylate (SAP).

2. The composition according to claim 1, **characterized in that** said superabsorbent polymer is present in an amount between 0.5 wt% and 8 wt%, preferably between 0.5 wt% and 5 wt% with respect to the total weight of the composition.

3. The composition according to Claim 1, **characterized in that** said at least one peroxide-based compound is selected from the group constituted by ammonium persulphate, potassium persulphate and sodium persulphate.

4. The composition according to Claim 1, **characterized in that** said at least one peroxide-based compound is present in an amount of between 10 wt% and 30 wt%, preferably between 20 wt% and 30 wt% with respect to the total weight of the composition.

5. The composition according to Claim 1, **characterized in that** said at least one alkalinizing agent is selected from the group constituted by sodium metasilicate and sodium silicate.

6. The composition according to Claim 1, **characterized in that** said at least one alkalinizing agent is present in an amount of between 5 wt% and 20 wt%, preferably between 10 wt.% and 15 wt% with respect to the total weight of the composition.

7. The composition according to any one of the preceding claims, **characterized in that** it further comprises magnesium carbonate.

8. Use of a superabsorbent polymer in an anhydrous powder composition for bleaching keratinic fibres, said composition comprising at least one peroxide-based compound, said superabsorbent polymer reducing the inflammability of said composition, wherein said superabsorbent polymer is a polymeric compound constituted by cross-linked sodium or potassium polymethacrylate (SAP)

9. Use of a superabsorbent polymer in an anhydrous powder composition for bleaching keratinic fibres, said composition comprising at least one peroxide-based compound, said superabsorbent polymer reducing the reactivity of a mixture obtained from the addition of an aqueous emulsion to said anhydrous composition, wherein said superabsorbent polymer is a polymeric compound constituted by cross-linked sodium or potassium polymethacrylate (SAP).

10. Use of a superabsorbent polymer in an anhydrous powder composition for bleaching keratinic fibres, said composition comprising at least one peroxide-based compound, said superabsorbent polymer increasing the stability of storage of said anhydrous composition, wherein said superabsorbent polymer is a polymeric compound constituted by cross-linked sodium or potassium polymethacrylate (SAP).

11. Use according to Claim 10, **characterized in that** said superabsorbent polymer maintains constant in time the titre of active oxygen of said at least one peroxide-based compound.

## Patentansprüche

1. Eine wasserfreie Pulverzusammensetzung zur Entfärbung von keratinischen Fasern, speziell von menschlichen keratinischen Fasern, bestehend aus mindestens einer peroxidbasierten Verbindung und mindestens einer alkalisierenden Substanz, **gekennzeichnet dadurch, dass** sie weiterhin ein superabsorbierendes Polymer enthält, wobei das superabsorbierende Polymer eine polymerische Verbindung, bestehend aus quervernetztem Natrium oder Kaliumpolymethacrylat (SAP), ist.

2. Die Zusammensetzung gemäß Anspruch 1, **dadurch gekennzeichnet, dass** das superabsorbierende Polymer in einem Umfang zwischen 0,5 Gew.-% bis 8 Gew.-% vorhanden ist, vorzugsweise zwischen 0,5 Gew.-% bis 5 Gew.-% in Bezug zum Gesamtgewicht der Zusammensetzung.

3. Die Zusammensetzung gemäß Anspruch 1, **gekennzeichnet dadurch, dass** mindestens eine peroxidbasierte Verbindung aus der Gruppe, bestehend aus Ammoniumpersulfat, Kaliumpersulfat und Natriumpersulfat, ausgewählt ist.

4. Die Zusammensetzung gemäß Anspruch 1, **gekennzeichnet dadurch, dass** mindestens eine peroxidbasierte Verbindung vorhanden ist, in einem Umfang von 10 Gew.-% bis 30 Gew.-%, vorzugsweise zwischen 20 Gew.-% bis 30 Gew.-% in Bezug zum Gesamtgewicht der Zusammensetzung.

5. Die Zusammensetzung gemäß Anspruch 1, **gekennzeichnet dadurch, dass** mindestens eine alkalisierende Substanz aus der Gruppe, bestehend aus Natriumsilikat und Natriummetasilikat, ausgewählt ist.

6. Die Zusammensetzung gemäß Anspruch 1, **dadurch gekennzeichnet, dass** mindestens eine alkalisierende Substanz vorhanden ist, in einem Umfang von 5 Gew.-% bis 20 Gew.-%, vorzugsweise zwischen 10 Gew.-% und 15 Gew.-% in Bezug zum Gesamtgewicht der Zusammensetzung.

7. Die Zusammensetzung gemäß irgendeinem der genannten Ansprüche, **dadurch gekennzeichnet, dass** sie weiterhin Magnesiumkarbonat umfasst.

8. Verwendung eines superabsorbierenden Polymers in einer wasserfreien Pulverzusammensetzung zur Entfärbung von keratinischen Fasern, wobei die Zusammensetzung mindestens eine peroxidbasierte Komponente enthält, wobei das superabsorbierende Polymer die Entzündlichkeit der Gesamtzusammensetzung reduziert, wobei das superabsorbierende Polymer eine polymerische Verbindung, bestehend aus quervernetztem Natrium oder Kaliumpolymethacrylat (SAP), ist.

9. Verwendung eines superabsorbierenden Polymers in einer wasserfreien Pulverzusammensetzung zur Entfärbung von keratinischen Fasern, wobei die Zusammensetzung mindestens eine peroxidbasierte Komponente enthält, wobei das superabsorbierende Polymer das Reaktionsvermögen der Mischung, erhalten durch das Hinzufügen einer wässrigen Emulsion zur wasserfreien Zusammensetzung, reduziert, wobei das superabsorbierende Polymer eine polymerische Verbindung, bestehend aus quervernetztem Natrium oder Kaliumpolymethacrylat (SAP), ist.

10. Verwendung eines superabsorbierenden Polymers in einer wasserfreien Pulverzusammensetzung zur Entfärbung von keratinischen Fasern, wobei die Zusammensetzung mindestens eine peroxidbasierte Komponente enthält, wobei das superabsorbierende Polymer die Beständigkeit der wasserfreien Zusammensetzung bei der Lagerung erhöht, wobei das superabsorbierende Polymer eine polymerische Verbindung, bestehend aus quervernetztem Natrium- oder Kaliumpolymethacrylat (SAP), ist.

11. Verwendung gemäß Anspruch 10, **dadurch gekennzeichnet, dass** das superabsorbierende Polymer den Titer von aktivem Sauerstoff von mindestens einer der peroxidbasierten Komponenten konstant hält.

## Revendications

1. Une composition de poudre anhydre pour blanchir des fibres kératiniques, en particulier des fibres kératiniques humaines, comprenant au moins un composé à base de peroxyde et au moins un agent alcalinisant, **caractérisée en ce qu'**elle comprend en outre un polymère superabsorbant, ledit polymère superabsorbant étant un composé polymère constitué de polyméthacrylate de sodium ou de potassium réticulé (SAP).

2. La composition de la revendication 1, **caractérisée en ce que** ledit polymère superabsorbant est présent en une quantité comprise entre 0,5 % en poids et 8 % en poids, de préférence entre 0,5 % en poids et 5 % en poids par rapport au poids total de la composition.

3. La composition de la revendication 1, **caractérisée en ce que** ledit au moins un composé à base de peroxyde est choisi dans le groupe constitué par le persulfate d'ammonium, le persulfate de potassium et le persulfate de sodium.

4. La composition de la revendication 1, **caractérisée en ce que** ledit au moins un composé à base de peroxyde est présent en une quantité comprise entre 10 % en poids et 30 % en poids, de préférence entre 20 % en poids et 30 % en poids par rapport au poids total de la composition.

5. La composition de la revendication 1, **caractérisée en ce que** ledit au moins un agent alcalinisant est choisi dans le groupe formé par le métasilicate de sodium et le silicate de sodium.

6. La composition de la revendication 1, **caractérisée en ce que** ledit au moins un agent alcalinisant est présent en une quantité comprise entre 5 % en poids et 20 % en poids, de préférence entre 10 % en poids et 15 % en poids par rapport au poids total de la composition.

7. La composition de l'une des revendications précédentes, **caractérisée en ce qu'**elle comprend en outre du carbonate de magnésium.

8. Utilisation d'un polymère superabsorbant dans une composition de poudre anhydre pour blanchir des fibres kératiniques, ladite composition comprenant au moins un composé à base de peroxyde, ledit polymère superabsorbant réduisant l'inflammabilité de ladite composition, ledit polymère superabsorbant étant un composé étant un composé polymère constitué de polyméthacrylate de sodium ou de potassium réticulé (SAP).

9. Utilisation d'un polymère superabsorbant dans une composition de poudre anhydre pour blanchir des fibres kératiniques, ladite composition comprenant au moins un composé à base de peroxyde, ledit polymère superabsorbant réduisant la réactivité d'un mélange obtenu par addition d'une émulsion aqueuse à ladite composition anhydre, ledit polymère superabsorbant étant un composé étant un composé polymère constitué de polyméthacrylate de sodium ou de potassium réticulé (SAP).

10. Utilisation d'un polymère superabsorbant dans une composition de poudre anhydre pour blanchir des fibres kératiniques, ladite composition comprenant au moins un composé à base de peroxyde, ledit polymère superabsorbant augmentant la stabilité de stockage de ladite composition anhydre, ledit polymère superabsorbant étant un composé étant un composé polymère constitué de polyméthacrylate de sodium ou de potassium réticulé (SAP).

11. Utilisation selon la revendication 10, **caractérisée en ce que** ledit polymère superabsorbant maintient constant dans le temps le titre de l'oxygène actif dudit au moins un composé à base de peroxyde.
